# EUROPEAN PATENT SPECIFICATION

(11) **EP 1 949 402 B1**
(45) Date of publication and mention of the grant of the patent: **13.04.2011**
(21) Application number: 06761095.6
(22) Date of filing: 27.06.2006
(51) Int. Cl.: H01J 5/50, F21V 23/06, A61L 2/10, H01J 61/36

(54) **Lamp device and radiation source assembly as well as water disinfection system comprising such lamp device**
Lampe und Strahlungsquelle sowie Wasserdesinfizierungsvorrichtung mit einer solchen Lampe
Lampe et source de rayonnement ainsi que système de désinfection de l'eau comprenant une telle lampe

(30) Priority: 24.06.2005 US 693502 P
(43) Date of publication of application: 30.07.2008
(73) Proprietor: TROJAN TECHNOLOGIES INC., London, Ontario N5V 4T7 (CA)
(72) Inventor: ELKU, Joseph, Tillsonburg, Ontario N4G 5V9 (CA); FRASER, Jim, St. Thomas, Ontario N5R 2H7 (CA); GRATTON, Richard, London, Ontario N6J 3V9 (CA)
(74) Representative: Hirsch & Associés
(86) International application number: PCT/CA2006/001040
(87) International publication number: WO 2006/136026

(56) References cited:
- EP-A2- 0 281 079
- JP-A- 62 115 625
- US-A- 5 166 527
- US-A1- 2002 168 881
- US-B1- 6 254 252
- US-B2- 6 488 510

## Description

### FIELD OF THE INVENTION

In one of its aspects, the present invention relates to a lamp device. In another of its aspects, the present invention relates to a radiation source assembly. In yet another of its aspects, the present invention relates to a water disinfection system.

### DESCRIPTION OF THE PRIOR ART

Fluid treatment systems such as water disinfection systems are generally known in the art.

See, for example, one or more of the following United States patents:
Re36,896,
3,418,370,
4,482,809,
4,872,980,
5,006,244,
5,471,063,
5,504,355,
5,538,210,
6,342,188,
6,500,346,
6,507,028,
6,646,269,
6,674,084,
6,803,586, and 6,863,078.

Document EP-A-0 281 079 discloses a low pressure discharge lamp includes a bulb with a pair of closed ends, and a pair of bases attached to the ends of the bulb, respectively. Each base has a cylindrical base body into which the end of bulb is inserted, and a pair of receiving terminals electrically connected to an electrode in the bulb and protruding from the base body in a direction perpendicular to the axis of the bulb.

Document JP-A-62 115 625 discloses a base for tubular bulb. A first member is formed through integral molding of the bottom of the bulb base, namely a bottom face and a terminal face upstanding from said bottom face. The terminal face is provided with four contact terminal pins projecting so that they may pierce the terminal face, while said pins being electrically connected to an external socket.

Many of the above-identified United States patents teach fluid treatment systems that employ ultraviolet (UV) radiation to kill, sterilize and/or prevent replication of microorganisms (bacteria, viruses, pathogens and the like) that may be present in the fluid.

Generally, such prior art fluid treatment systems employ an ultraviolet radiation lamp to emit radiation of a particular wavelength or range of wavelengths (usually between 185 and 400 nm) to kill, sterilize and/or prevent replication of microorganisms (bacteria, viruses, pathogens and the like) that may be present in the fluid.

Conventional ultraviolet radiation lamps include low pressure lamps, medium pressure lamps, low pressure high output lamps and the like.

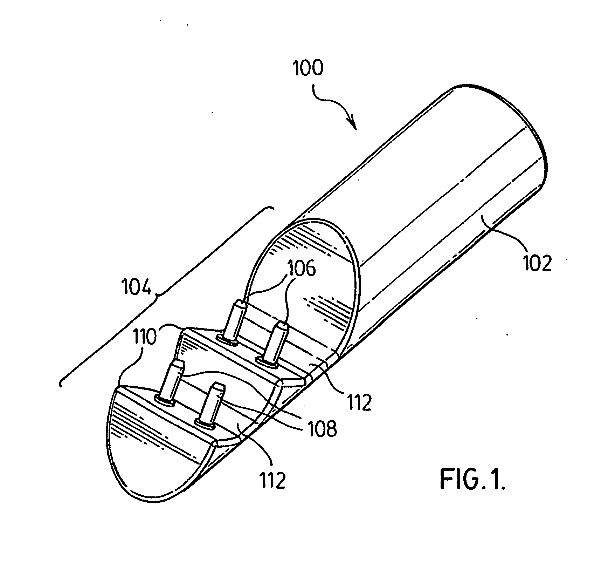

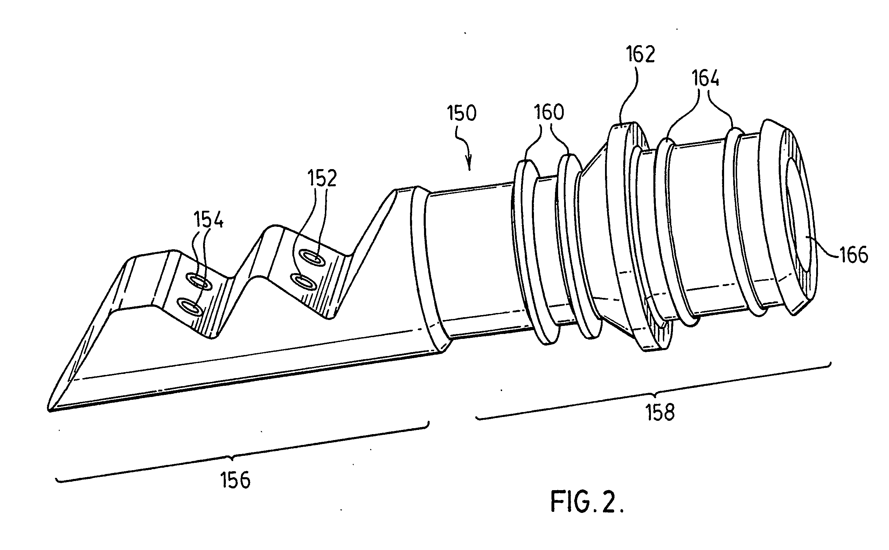

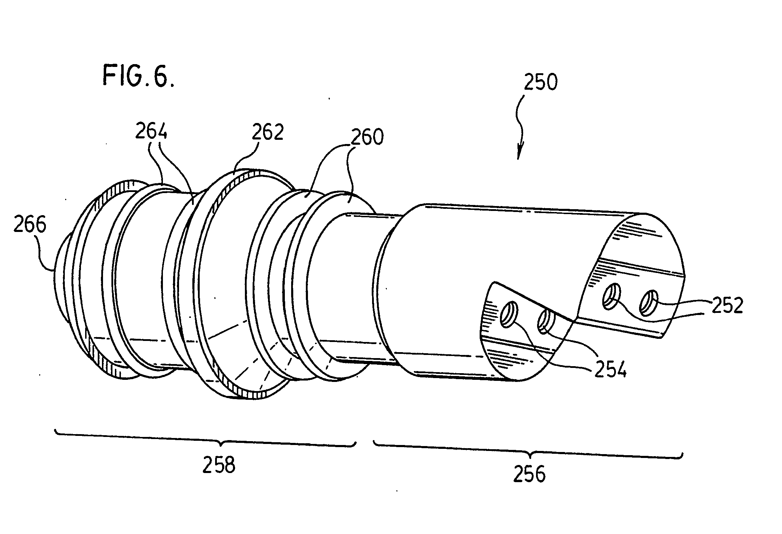

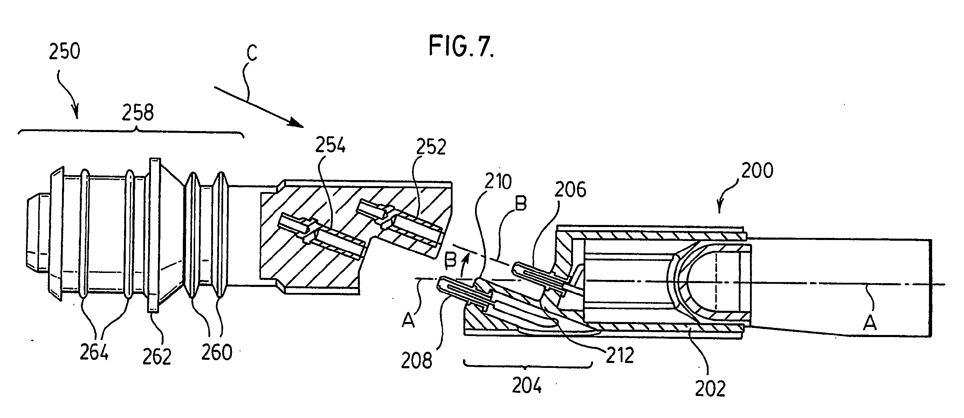

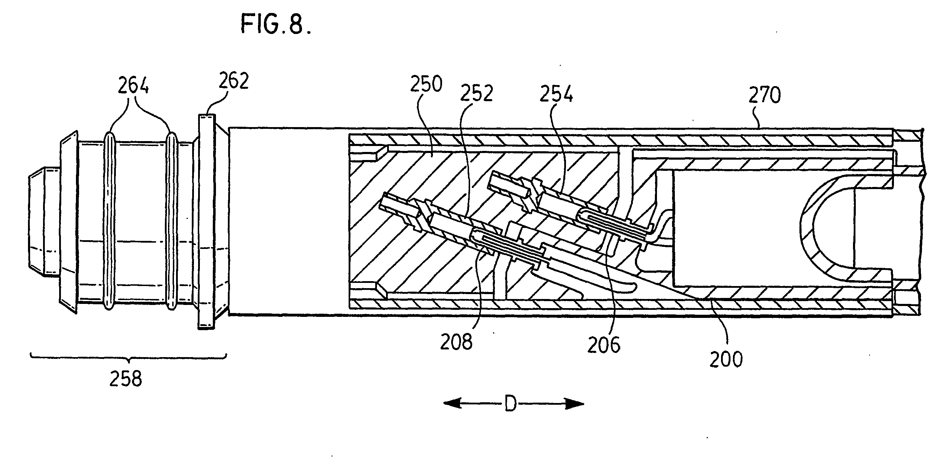

In more recent years, it has become conventional to use such ultraviolet lamps configured to have all of the electrical connections disposed at one end of the lamp. See, for example, Figures 2-8 of United States patent 4,700,101 [Ellner et al. (Ellner)] and Figures 1, 2 and 4 of United States patent 5,166,527 [Solymar].

As can be seen from the prior art radiation lamps taught by Ellner and Solymar, the electrical connection pins are elongate and are disposed such that the axes of the pins are parallel with the longitudinal axes of radiation lamp. In other words, the electrical connection is made by pushing an end cap or other connection base on to the pins in a direction parallel to the longitudinal axis of the radiation lamp.

The problem with this approach is that in many applications, the radiation lamp is immersed in a flow of water and turbulence created within that water treatment system invariably imparts a vibratory motion to the lamps which frequently results in lamps being vibrated or shaken loose of its electrical connection base or socket thereby causing the lamps to be rendered completely or intermittently inoperative. When such an event occurs, the water being treated may not be fully disinfected.

More recently, the prior art has attempted to address this problem by using a relatively complicated mechanical connection (e.g., a so-called "push-and-twist" connection) to secure the lamp to the connection base. See, for example, United States patent 5,422,487 [Sauska et al (Sauska)] and United States patent 6,884,103 [Kovacs]. The problem with these approaches is the complexity of the mechanical connection between the lamp and the base unit requiring the use of springs, specialized connection lugs and the like. Further, a connection system which is predicated on a dual motion system such that pushing and twisting gives rise to a higher incidents of lamp breakage and other damage to the module by field personal.

Accordingly, there remains the need in the art for a lamp device, particularly a radiation lamp, which will provide a reliable electric connection on the one hand, yet be relatively inexpensive, uncomplicated and simple to implement on the other hand.

### SUMMARY OF THE INVENTION

It is an object of the present invention to obviate or mitigate at least one of the above-mentioned disadvantages of the prior art.

Accordingly, in one of its aspects, the present invention provides a lamp device according to claim 1.

In another of its aspects, the present invention relates to a radiation source assembly comprising such a lamp device, together with a radiation transparent protective sleeve.

Other aspects of the present invention relate to water disinfection systems incorporating the above lamp device, and radiation source assembly, respectively.

### BRIEF DESCRIPTION OF THE DRAWINGS

Embodiments of the present invention will be described with reference to the accompanying drawings, wherein like reference numerals denote like parts, and in which:
Figures 1-4 illustrate components of a first preferred embodiment of the present lamp device; and
Figures 5-8 illustrate portions of a second preferred embodiment of the present lamp device.

### DETAILED DESCRIPTION OF TIRE PREFERRED EMBODIMENTS

Preferred embodiments of the present invention will be described below with reference to Figures 1-8. In Figures 1-8, there is shown in detail the electrical connection portion of a radiation lamp and/or the connection thereof to a complementary base unit. The remaining detail of the lamp is not shown for easier understanding. However, those of skill in the art will readily recognize how the specific connection shown in the Figures can be implemented in a lamp design by reference to the various prior art patents referred to above.

With reference to Figures 1-4, there is illustrated a first electrical base unit 100 and a second electrical base unit 150.

First electrical connection base unit 100 comprises a housing 102. An end portion 104 of unit 100 comprises a first pair of electrical connectors 106 and a second pair of electrical connectors 108.

In portion 104 comprises an undulating shape having a series of peaks 110 and valleys 112. Electrical connectors 106,108 are connected to wire or other suitable electrical conveyance (not shown) disposed within housing 102 which is than connected to the radiation-emitting cavity of a lamp in a conventional manner.

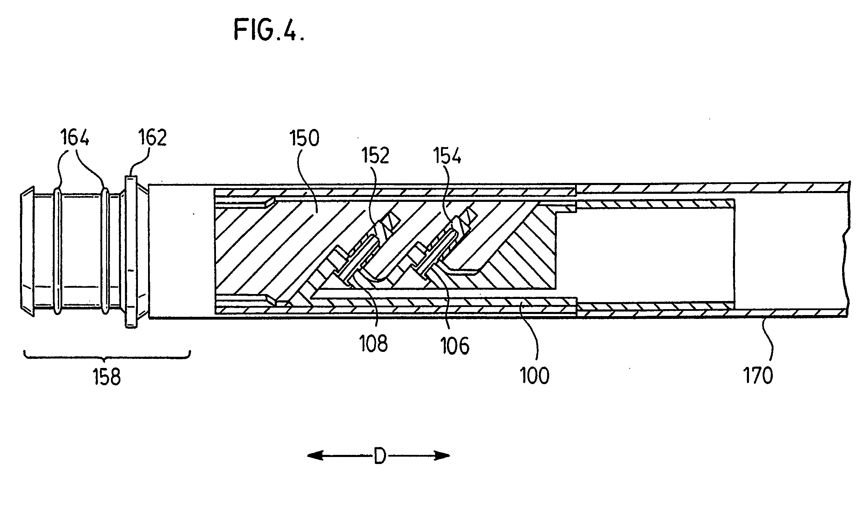

With particular reference to Figure 2, there is illustrated an enlarged view of base unit 150. As shown, base unit 150 comprises a first pair of electrical receptacles 152 and a second pair of electrical receptacles 154. These receptacles are disposed at a first end portion 156 of base unit 150. Disposed at another end portion 158 of base unit 150 is a sealing system which functions in a manner similar to that described in United States patents 4,872,980 and 5,006,244 referred to above. Specifically, end portion 158 comprises a pair of annular seals 160 that serve to seal the interior of a radiation transparent protective sleeve (Figure 4) placed over the radiation lamp.

Also disposed on end portion 158 is a stop portion 162 that serves as stop for the open end of protective sleeves surrounding the lamp.

Portion 158 further comprises a pair of annular seals 164 that serve to seal against water ingress into the frame of a module in which the lamp is placed (again, reference can be made to United States patent 4,872,980 and 5,006,244 for further details on the function of the seals).

Electrical receptacles 152,154 are wired in a conventional manner with the electrical wires or other electrical conveyance emerging from an aperture 166 in end portion 158 connection unit 150.

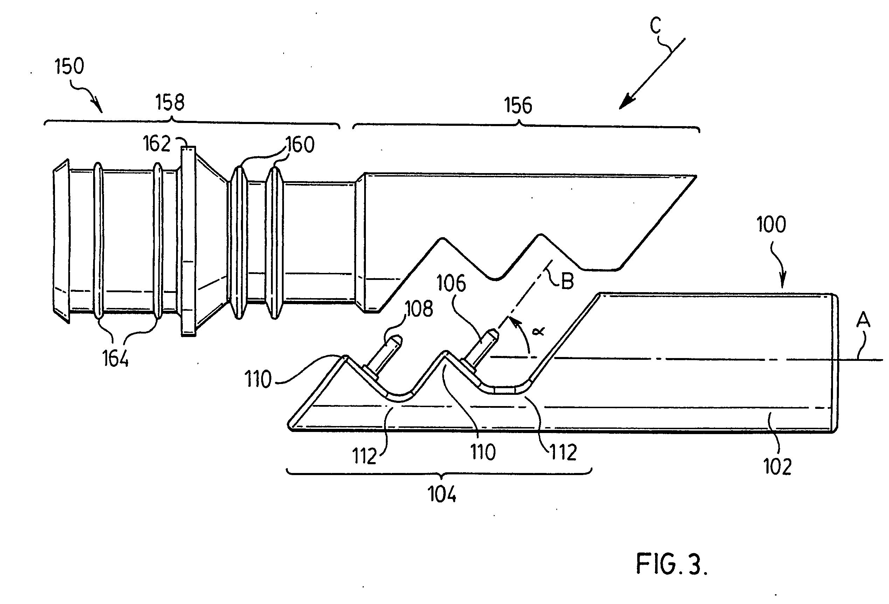

With particular reference to Figure 3, it will be seen that when base unit 100 is connected to the remaining components of the radiation lamp, there will be a longitudinal axes shown at line A through base unit 100 and the remainder of the lamp. Further, it will be seen that electrical connector 106,108 are elongate and have a longitudinal axis through line B. An important feature of this preferred embodiment is that line B is non-parallel with respect to line A. In other words, the longitudinal axes through electrical connectors 106,108 is in a non-parallel relationship with the longitudinal axes through base unit 100 and the remainder of the radiation lamp. In the specific embodiment shown, electrical connectors 106,108 are disposed at an acute angle α toward the radiation-emitting cavity (not shown) of the lamp.

When it is desired to engage base unit 100 with base unit 150, the base units are aligned as shown in Figure 3 and moved with respect to one another in the direction of arrow C. The engaged components are shown in Figure 4 and a radiation transparent protective sleeve 170 encases the connected units 100 and 150. As will be appreciated by those of skill in the art, once the connection is shown as made in Figure 4, the connection will withstand vibration forces conveyed to the radiation lamp. Specifically, base unit 100 will not separate from base unit 150 in a direction of arrow D, particularly when radiation transparent protective sleeve 170 is disposed over the connection.

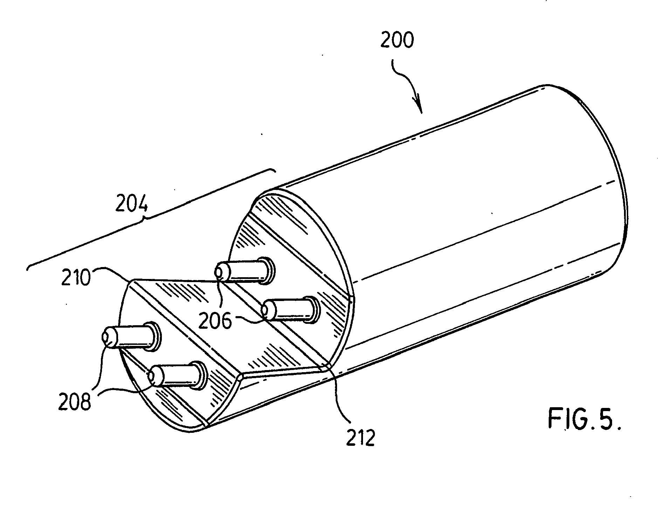

With reference to Figures 5-8, there is illustrated a second embodiment of the preferred invention. In the description of Figures 5-8, reference numerals will be used wherein the last two digits correspond to the same relevance appearing in Figures 1-4 and the first numeral will be "2" in Figures 5-8 instead of "1" in Figures 1-4. Thus, it will be seen that a first major difference between the two embodiments is that the embodiment illustrated in Figures 5-8 comprises electrical connectors 206,208 that are angled away (β) from the radiation-emitting cavity. Another difference is that the cross-sectional shape base unit 200 in Figures 5-8 comprises a single valley 212 instead of the pair of valleys 112 in the embodiment illustrated in Figures 1-4. Yet another difference is that the cross-sectional shape base unit 200 in Figures 5-8 comprises a single peak 210 instead of the pair of valleys 110 in the embodiment illustrated in Figures 1-4. Otherwise, the embodiment shown in Figures 5-8 may be used in a manner similar to that described above with respect to Figures 1-4.

While this invention has been described with reference to illustrative embodiments and examples, the description is not intended to be construed in a limiting sense. Thus, various modifications of the illustrative embodiments, as well as other embodiments of the invention, will be apparent to persons skilled in the art upon reference to this description. For example, it is possible to reverse the arrangement of electrical connectors and electrical receptacles in the illustrated embodiments - i.e., the electrical connectors would be disposed in electrical connection base unit 150,250 and the electrical receptacles would be disposed in electrical connection base unit 100,200. Also, it is possible to have a mixture of electrical connectors and electrical receptacles on electrical connection base unit 100,200 and a complementary mixture of electrical connectors and electrical receptacles on electrical connection base unit 150,250. Yet another modification of the illustrated embodiments relates to the use of electrical connection bases that, in cross-section contain no peaks or valleys. It is therefore contemplated that the appended claims will cover any such modifications or embodiments.

## Claims

1. A lamp device comprising an elongated radiation emitting cavity having a longitudinal axis (A), a first elongate electrical connector (106) and a second elongate electrical connector (108), each of the first elongate electrical connector and the second elongate connector being:
(i) non-parallel with respect to the longitudinal axis, and
(ii) disposed in a base portion (104) at one end of the lamp device,
**characterized in that** the first elongate electrical connector and the second elongate connector are disposed at an acute angle (α, β) with respect to the longitudinal axis (A).

2. The lamp device defined in Claim 2, wherein the first elongate electrical connector and the second elongate electrical connector are substantially parallel with respect to one another.

3. The lamp device defined in any one of Claims 1-2, wherein the first elongate electrical connector and the second elongate connector are in a spaced relationship along the longitudinal axis.

4. The lamp device defined in any one of Claims 1-2, wherein the first elongate electrical connector and the second elongate electrical connector are aligned in a spaced relationship along the longitudinal axis.

5. The lamp device defined in Claim 4, wherein a cross-section of the base portion containing the longitudinal axis comprises an undulating shape.

6. The lamp device defined in Claim 4, wherein a cross-section of the base portion containing the longitudinal axis comprises at least one peak and at least one valley.

7. The lamp device defined in Claim 6, wherein the first elongate electrical connector and the second elongate electrical connector are offset with regard to the at least one peak or the at least one valley.

8. The lamp device defined in Claim 6, wherein the first elongate electrical connector and the second elongate electrical connector are offset with regard to both of the at least one peak and the at least one valley.

9. The lamp device defined in Claim 4, wherein a cross-section of the base portion containing the longitudinal axis comprises a single peak and a single valley.

10. The lamp device defined in any one of Claims 1-9, wherein both of the first elongate electrical connector and the second elongate electrical connector comprises a male portion of a male-female connection system.

11. The lamp device defined in any one of Claims 1-10, comprising a pair of first elongate electrical connectors and a pair of second elongate electrical connectors.

12. The lamp device defined in Claim 11, wherein the pair of first elongate electrical connectors are in substantial alignment along a line orthogonal to the longitudinal axis.

13. The lamp device defined in Claim 11, wherein the pair of second elongate electrical connectors are in substantial alignment along a line orthogonal to the longitudinal axis.

14. The lamp device defined in Claim 11, wherein the pair of first elongate electrical connectors and the pair of second elongate electrical connectors are in substantial alignment along a line orthogonal to the longitudinal axis.

15. A radiation source assembly comprising the lamp device defined in any one of Claims 1-14, together with a radiation transparent protective sleeve.

16. A water disinfection system comprising the lamp device defined in any one of Claims 1-14.

## Patentansprüche

1. Lampe mit einem lang gestreckten strahlungsemittierenden Hohlraum, der eine Längsachse (A), einen ersten lang gestreckten elektrischen Anschluss (106) und einen zweiten lang gestreckten elektrischen Anschluss (108) aufweist, wobei der erste und der zweite elektrische Anschluss
(i) nicht parallel zur Längsachse, und
(ii) in einem Basisteil (104) an einem Ende der Lampe angeordnet sind,
**dadurch gekennzeichnet, dass** der erste lang gestreckte elektrische Anschluss und der zweite lang gestreckte elektrische Anschluss unter einem spitzen Winkel (α, β) in Bezug auf die Längsachse (A) angeordnet sind.

2. Lampe nach Anspruch 1, **dadurch gekennzeichnet, dass** der erste lang gestreckte elektrische Anschluss sowie der zweite lang gestreckte elektrische Anschluss im Wesentlichen parallel zueinander verlaufen.

3. Lampe nach einem der Ansprüche 1 bis 2, **dadurch gekennzeichnet, dass** der erste lang gestreckte elektrische Anschluss und der zweite lang gestreckte elektrische Anschluss entlang der Längsachse einen gegenseitigen Abstand aufweisen.

4. Lampe nach einem der Ansprüche 1 oder 2, **dadurch gekennzeichnet, dass** der erste lang gestreckte elektrische Anschluss und der zweite lang gestreckte elektrische Anschluss entlang der Längsachse in einen gegenseitigen Abstand miteinander fluchten.

5. Lampe nach Anspruch 4, **dadurch gekennzeichnet, dass** der Querschnitt des Basisteiles, der die Längsachse umfasst, eine gewellte Gestalt hat.

6. Lampe nach Anspruch 4, **dadurch gekennzeichnet, dass** der Querschnitt des Basisteiles, der die Längsachse umfasst, wenigstens einen Gipfel und wenigstens ein Tal umfasst.

7. Lampe nach Anspruch 6, **dadurch gekennzeichnet, dass** der erste lang gestreckte elektrische Anschluss und der zweite lang gestreckte elektrische Anschluss in Bezug auf wenigstens eines der beiden, Gipfel oder Tal, gegeneinander versetzt sind.

8. Lampe nach Anspruch 6, **dadurch gekennzeichnet, dass** der erste lang gestreckte elektrische Anschluss und der zweite lang gestreckte elektrische Anschluss in Bezug auf Gipfel und Tal versetzt sind.

9. Lampe nach Anspruch 4, **dadurch gekennzeichnet, dass** ein Querschnitt des Basisteiles, der die lang gestreckte Achse umfasst, einen einzigen Gipfel und ein einziges Tal aufweist.

10. Lampe nach einem der Ansprüche 1 bis 9, **dadurch gekennzeichnet, dass** die beiden lang gestreckten elektrischen Anschlüsse ein männliches und ein weibliches Verbindungssystem umfassen.

11. Lampe nach einem der Ansprüche 1 bis 10, umfassend ein Paar erster und zweiter lang gestreckter elektrischer Anschlüsse.

12. Lampe nach Anspruch 11, **dadurch gekennzeichnet, dass** das Paar erster lang gestreckter elektrischer Anschlüsse im Wesentlichen in einer Linie rechtwinklig zur Längsachse verläuft.

13. Lampe nach Anspruch 11, **dadurch gekennzeichnet, dass** das Paar zweiter lang gestreckter elektrischer Anschlüsse im Wesentlichen entlang einer Linie rechtwinklig zur Längsachse verläuft.

14. Lampe nach Anspruch 11, **dadurch gekennzeichnet, dass** das erste und das zweite Paar lang gestreckter elektrischer Anschlüsse im Wesentlichen entlang einer Linie senkrecht zur Längsachse verlaufen.

15. Strahlenquelle, umfassend die Lampe gemäß einem der Ansprüche 1 bis 14, zusammen mit einer strahlungstransparenten Schutzhülse.

16. Wasserdesinfektionssystem, umfassend die Lampe gemäß einem der Ansprüche 1 bis 14.

## Revendications

1. Un dispositif de lampe comprenant une cavité allongé émettant un rayonnement présentant un axe longitudinal (A), un premier connecteur électrique allongé (106) et un deuxième connecteur électrique allongé (108), chacun parmi le premier connecteur électrique allongé et le deuxième connecteur allongé étant :
(i) non-parallèle par rapport à l'axe longitudinal, et
(ii) disposé dans une partie de base (104) à une extrémité du dispositif de lampe,
**caractérisé en ce que** le premier connecteur électrique allongé et le deuxième connecteur allongé sont disposés selon un angle aigu (α, β) par rapport à l'axe longitudinal (A).

2. Le dispositif de lampe selon la revendication 1, dans lequel le premier connecteur électrique allongé et le deuxième connecteur électrique allongé sont sensiblement parallèles l'un par rapport à l'autre.

3. Le dispositif de lampe selon l'une des revendications 1-2, dans lequel le premier connecteur électrique allongé et le deuxième connecteur allongé présentent une relation espacée le long de l'axe longitudinal.

4. Le dispositif de lampe selon l'une des revendications 1-2, dans lequel le premier connecteur électrique allongé et le deuxième connecteur électrique allongé sont alignés dans une relation espacée le long de l'axe longitudinal.

5. Le dispositif de lampe selon la revendication 4, dans lequel une section transversale de la partie de base contenant l'axe longitudinal comprend une forme ondulée.

6. Le dispositif de lampe selon la revendication 4, dans lequel une section transversale de la partie de base contenant l'axe longitudinal comprend au moins une crête et au moins une vallée.

7. Le dispositif de lampe selon la revendication 6, dans lequel le premier connecteur électrique allongé et le deuxième connecteur électrique allongé sont décalés par rapport à l'au moins une crête ou à l'au moins une vallée.

8. Le dispositif de lampe selon la revendication 6, dans lequel le premier connecteur électrique allongé et le deuxième connecteur électrique allongé sont décalés par rapport à l'au moins une crête et à l'au moins une vallée.

9. Le dispositif de lampe selon la revendication 4, dans lequel une section transversale de la partie de base contenant l'axe longitudinal comprend une seule crête et une seule vallée.

10. Le dispositif de lampe selon l'une des revendications 1-9, dans lequel le premier connecteur électrique allongé ainsi que le deuxième connecteur électrique allongé comprennent une partie mâle d'un système de connexion mâle-femelle.

11. Le dispositif de lampe selon l'une des revendications 1-10, comprenant une paire de premiers connecteurs électriques allongés et une paire de deuxièmes connecteurs électriques allongés.

12. Le dispositif de lampe selon la revendication 11, dans lequel la paire de premiers connecteurs électriques allongés sont sensiblement en alignement le long d'une ligne orthogonale à l'axe longitudinal.

13. Le dispositif de lampe selon la revendication 11, dans lequel la paire de deuxièmes connecteurs électriques allongés sont sensiblement en alignement le long d'une ligne orthogonale à l'axe longitudinal.

14. Le dispositif de lampe selon la revendication 11, dans lequel la paire de premiers connecteurs électriques allongés et la paire de deuxièmes connecteurs électriques allongés sont sensiblement en alignement le long d'une ligne orthogonale à l'axe longitudinal.

15. Un assemblage de source de rayonnement comprenant le dispositif de lampe selon l'une quelconque des revendications 1-14, avec une gaine de protection transparente au rayonnement.

16. Un système de désinfection d'eau comprenant le dispositif de lampe selon l'une quelconque des revendications 1-14.
